Europäisches Patentamt

European Patent Office

Office européen des brevets

(19)

(11) Publication number: **0 141 581**
A2

(12) # EUROPEAN PATENT APPLICATION

(21) Application number: **84307145.7**

(22) Date of filing: **17.10.84**

(51) Int. Cl.⁴: **G 01 N 33/543**, G 01 N 33/58

(30) Priority: **21.10.83 US 544307**

(43) Date of publication of application: **15.05.85**
**Bulletin 85/20**

(84) Designated Contracting States: **AT BE CH DE FR GB IT LI LU NL SE**

(71) Applicant: **HEMOGENETICS, INC., 101 South San Mateo Drive Suite 315, San Mateo California 94401 (US)**

(72) Inventor: **Der-Ballan, Georges P., 505 Cypress Point Drive, No. 206, Mountain View California 94043 (US)**
Inventor: **Jaeger, Frederick, 2737 Elmwood, Berkeley California 94705 (US)**
Inventor: **Gullford, Frederick T., 829 Forest Avenue, Palo Alto California 94301 (US)**

(74) Representative: **Paget, Hugh Charles Edward et al, MEWBURN ELLIS & CO. 2/3 Cursitor Street, London EC4A 1BQ (GB)**

(54) Labelling system for specific binding assays.

(57) A highly sensitive bioluminescent assay employing a biotinylated firefly luciferase label is disclosed. The assay protocol utilizes biotin and avidin as intermediate binding substances. In the preferred embodiment, total IgE or specific IgE are reacted with immobilized anti-(IgE) or immobilized specific allergen, respectively. The solid phase is next reacted with biotinylated anti-(IgE), and the amount of binding determined by exposure to free avidin and biotinylated firefly luciferase.

EP 0 141 581 A2

9894-1/HHHHH2

# LABELLING SYSTEM FOR SPECIFIC BINDING ASSAYS

## Background of the Invention

### 1. Field of the Invention

The present invention relates generally to specific binding assays employing enzyme labels, and more particularly to a novel labelling system employing biotinylated firefly luciferase.

A wide variety of specific binding assays are known. Such assays may be heterogeneous or homogeneous, competitive or noncompetitive, and may employ any one of a number of labels, including radioisotopes, enzymes, fluorescers, chemiluminescers, bioluminescers, and other detectable molecules.

When devising an assay protocol, it is critical that all aspects of the protocol be able to function together. This is a particular concern when developing an assay employing an enzyme label since enzyme activity can be inhibited by many factors, including conjugation of the enzyme to a specific binding protein or ligand, as well as by steric inhibition which occurs during the binding reactions in the assay itself. For these reasons, it is never possible to predict with certainty whether a particular enzyme label can be adapted to assay protocols where it has not been used previously. Moreover, it is highly desirable that modified enzyme labelling reagents remain stable over relatively long periods, on the order of months rather than days, so that the reagents can be prepared in advance and distributed in kits which may be utilized sometime later.

Firefly luciferase offers a number of advantages when used as a label in a specific binding assays. The enzyme may be detected substantially instantaneously in the presence of appropriate substrates, in contrast to other enzymes which require the accumulation of product over time for detection. Moreover, very low levels of firefly luciferase, on the order of $10^{-16}$M, and below, may be detected. Such sensitivity can lower the cost of the assay since reagent usage can be reduced. Firefly luciferase, however, is easily inactivated when conjugated to other molecules and has not heretofore enjoyed widespread use as a labelling reagent in specific binding assays.

Certain assay protocols have been developed which utilize biotin and avidin as intermediate binding compounds. Typically, a biotinylated primary reagent specific for the antigen of interest is first reacted with the sample. The sample can either be a liquid assay medium containing solubilized antigen or a solid phase on which the antigen has been immobilized. After the reaction is complete, the primary reagent bound to the antigen may be separated from the reaction medium and thereafter reacted with labelled avidin. Alternatively, the primary reagent can be unlabelled and a secondary biotinylated reagent capable of binding the primary reagent utilized. Labelled avidin is then reacted with the secondary reagent. This latter method provides for amplification of the label and allows the use of a common biotinylated reagent for detection of a number of different antigens.

It would be desirable to provide an assay which combines the advantages of using a firefly luciferase label with those of using biotin and avidin as intermediate labelling substances. In particular, it would be desirable to provide a biotinylated luciferase which retains a high percentage of its activity for use in such assays.

## 2.    Description of the Prior Art

Specific binding assays using firefly luciferase as a label are known.  See, e.g., U.S. Patent No. 4,380,580, and Schroeder et al. (1976) Anal. Chem. 48:1933-1937.  Specific binding assays using biotin and avidin as intermediate binding substances are known.  See, e.g., U.S. Patent No. 4,228,237.  The use of biotin and avidin in enzymatic histochemical staining is known.  See, e.g., Hsu et al. (1981) J. Histo. Chem. Cytochem. 29:577-580, and Vector Laboratories, Inc., Burlingame, California, 1982 Catalogue, pp. 8-9.  A method for conjugating firefly luciferase to particular antigens has been taught by Wannlund et al. (1980) Biochem. Biophys. Res. Comm. 96:440-446, and Wannlund and DeLuca (1983) Methods In Enzymology 92:426-432.  Assays for determining total and specific IgE are known.  See, e.g., U.S. Patents Nos. 4,347,311 and 4,292,403.

## Summary of the Invention

The present invention provides a heterogeneous immunoassay employing a biotin-firefly luciferase conjugate (biotinylated firefly luciferase) as a label.  The biotinylated firefly luciferase is characterized by retention of at least 50% of the activity of the unmodified luciferase.  The assay generally detects antigen which has been immobilized onto a solid phase, typically by reaction with an immobilized receptor specific for the antigen. Biotinylated firefly luciferase and avidin are then reacted with exposed solid phase in a predetermined order.  The biotinylated luciferase can be detected by reaction with the appropriate substrate and the amount of antigen present in the sample determined based on the amount of bound or unbound luciferase.

4

## Description of the Specific Embodiments

The present invention is suitable for quantitating the amount of bound ligand or antigen immobilized on a solid phase. Typically, the ligand will be immobilized by reaction between a first receptor specific for the ligand bound to the solid phase and a liquid assay medium suspected of containing the ligand. By providing excess first receptor, the entire amount of ligand present in the sample can be bound. After the solid phase is removed from the assay medium, it is reacted with a second receptor which has been conjugated to a plurality of biotin moities. The second receptor will be specific for the ligand and may be the same substance or a different substance than the first receptor. Excess second receptor is provided so that all bound ligand is reacted. The solid phase is further reacted with free avidin and biotinylated firefly luciferase to form bridged labelling complexes. The biotinylated second receptor, avidin and biotinylated firefly luciferase may be combined sequentially or simultaneously, or the avidin and biotinylated firefly luciferase may be preformed into a complex and reacted with the solid phase subsequent to reaction with the biotinylated second receptor.

Although the present invention is suitable for detection of any ligand which can be immobilized on a solid phase, it is particularly useful for the determination of total IgE and specific IgE in the blood. IgE is present in normal serum at very low concentrations on the order of $10^{-5}$ to $10^{-4}$ mg per ml. The detection of elevated serum IgE levels can be useful in the diagnosis of a patient's allergic response. The sensitivity of the present assay makes it particularly suitable for the determination of IgE levels. Moreover, firefly luciferase, unlike other enzyme labels obtained from bacterial and other sources, will almost certainly not be present in human sera and will not therefore interfere with the assay or provide undesirable background.

In the exemplary IgE assays, the first receptor will be either anti-IgE (for determining total IgE) or a particular allergen characteristic of an IgE class, i.e., particular grasses, trees, drugs, microbial antigens, food antigens, and the like. Anti-(human IgE) can be obtained commercially from a number of sources, including Tago, Burlingame, California.

Particular antibodies against allergens which might be detected by the assay of the present invention include grasses, such as bent grass, perennial rye grass, and the like; dander, such as dog dander, cat dander, and the like; foods, such as apple, spinach, shrimp, and the like; insect venom, such as mosquito venom, wasp venom, bee venom, and the like; trees, such as elm, oak, acacia, and the like; bacteria, such as Diplococcus, Pneumoniae, and the like; viruses, such as hepatitis virus, and the like; flowers, such as marigold, sunflower, and the like; and weeds, such as pigweed, ragweed, Russian thistle, and the like. This is only a partial list, and it will be appreciated that many other allergens may be detected in serum using the assay of the present invention.

As stated above, the first receptor (either anti-IgE or allergen) will be immobilized to a solid carrier or solid phase by conventional techniques. The solid phase may be any water-soluble solid carrier where coupling may be effected by means of covalent bonds, adsorption, or the like. In particular, the solid phase may comprise beads, strips, test tubes, microtiter plates, and other constructions which facilitate exposure to the serum sample.

The solid phase support of the present invention may be constructed from a wide variety of materials and need not be formed from a single material. In the broadest sense, it is necessary only that the material selected be capable of being formed into the desired geometry, be insoluble in the assay medium, and have the ability to covalently or non-covalently bond the first receptor without otherwise interfering with the binding between the first receptor and the ligand. Preferred are synthetic polymeric resins, such as polyacrylamide, polystyrene, polyethylene, polypropylene, polyvinyl compounds such as polyvinyl chloride, polyacrylate, polymethacrylate, and co-polymers thereof.

Immobilization of the first receptor can be achieved by a wide variety of known techniques. Depending on the nature of the particular material, as well as on the nature of the receptor (i.e., IgE or allergen) physical absorption or adsorption may be employed. Preferably, the first receptor will be covalently bonded to the surface of the solid phase support. The surface can be activated or spacer arms can be covalently bonded to the surface by various techniques well known in the art, leaving a functionality which is either active or can be activated to react with the receptor. For example, nitriles can be modified to form imido esters, which will react with the available amino groups on the antibody or antigen. There is extensive literature concerning such bonding of proteins to surfaces employing activated carboxylic acids, carbodiimides, imido esters, active alkyl halides, etc., to form amido, amide, or amino linkages.

To immobilize antigens, particularly allergens, it is preferred to use glutaraldehyde both to activate the solid phase and to cross-link the allergens as they are bound to the solid phase. Any solid phase having available amino groups, such as polyacrylamide, polystyrene, polypropylene, and the

like, or which may be modified to have amino groups, such as nylon, many be activated with glutaraldehyde. Conveniently, the solid phase is reacted with about 1% glutaraldehyde in a basic buffer (e.g., 0.1M carbonate, pH 9.0) and reacted at an elevated temperature for about 1-2 hours. After a thorough washing, the solid phase is reacted with the allergen, typically diluted about 1:8 in PBS, in the presence of about 0.01 - 0.1% glutaraldehyde by weight. The glutaraldehyde promotes cross-linking of the allergens as they bind to the activated amino groups on the solid phase. The reaction is carried out in the cold (4°C) for several hours, preferably overnight. Solid phases prepared by this technique are found to have improved binding capacities when compared to those prepared by conventional techniques.

The biotinylated second receptor can be obtained commercially or prepared by conventional techniques. In the exemplary assays, the second receptor will be anti-(human IgE), and both the unmodified antibody and the biotinylated antibody are available from commercial sources, such as Tago and Vector Laboratories, Inc., Burlingame, California. Alternatively, antibodies can be biotinylated by conventional techniques. Typically, antibody is reacted with a biotin derivative, such as a biotin ester, i.e., N-hydroxysuccinimide ester (Biosearch, San Rafael, California). The biotin ester is dissolved in a polar, aprotic solvent, such as dimethylformamide, and is combined in molar excess with the antibody. The mixture is allowed to react at a low temperature, typically from 2 to 10°C for a number of hours. After completion of the reaction, the biotinylated antibody may be separated by known techniques. Particular methods for preparing biotinylated antibodies are taught in U.S. Patent No.

8

4,298,685, the disclosure of which is incorporated herein by reference.

The avidin utilized in the method of the present invention is available from a number of commercial sources, including Vector Laboratories, Inc., Burlingame, California.

The biotinylation of the firefly luciferase is critical. The firefly luciferase must retain at least 50% of the activity of the unmodified enzyme, preferably at least 60% or greater. Firefly luciferase may be obtained from commercial vendors, such as Sigma Chemical Company, St. Louis, Missouri. The firefly luciferase may be combined with N-hydroxysuccinimide biotin ester in a phosphate buffer to achieve linkage through the amino groups on the enzyme. The weight ratio of firefly luciferase to biotin ester should be in the range from about 1:1 to 1:10, preferably in the range from about 1:4 to 1:8 (luciferase:biotin ester, molar ratio). The luciferase and biotin ester should be combined in the presence of substrate, or a substrate analog, in order to protect the active site of the enzyme during derivatization. Conveniently, adenosine triphosphate should be present at about 5mM. Biotinylated firefly luciferase prepared in this manner retains a high percentage (above 50%) of its activity for extended periods.

For the most part, the method of the invention will be used to quantitate the amount of ligand or antigen present in a biological sample such as blood, serum, urine, or solid tissue. Methods for preparing such biological samples for immunological assays are well known. Blood and serum samples will be mixed with an appropriate buffer prior to combination with the remaining assay reagents. For the exemplary IgE assays, it will often be desirable to dilute the blood sample to lower the concentration of IgE to within the useful range of the assay. In particular, it has been found that by including a combination of detergents in the assay medium, preferably 0.1% Tween® and 0.1% Triton® X-100 by weight, the background light emission is greatly reduced. In the case of solid biological specimens, it will be necessary to solubilize the specimen in a liquid assay medium.

Once the liquid assay medium has been prepared, it will be incubated with the immobilized first receptor (solid phase) until equilibrium has been reached. For the binding of IgE to its specific allergen or to anti-(human IgE), this will usually take from one to two hours at 37°C. After separating the assay medium, the solid phase is washed to remove all unbound antibodies. The solid phase is next reacted with the biotinylated second receptor, e.g., biotinylated anti-(human IgE). In this way, biotin is immobilized on the solid phase in direct proportion to the amount of IgE or specific IgE in the sample. To complete the labelling, the solid phase is reacted with free avidin and the biotinylated firefly luciferase in a predetermined order. Preferably, the avidin and firefly luciferase are added sequentially or simultaneously. Alternatively, the avidin and firefly luciferase may be pre-reacted to form a labelling complex prior to reaction with the solid phase. In either case, the weight ratio of avidin to biotinylated firefly luciferase should be in the range from about 10:1 to 100:1 (avidin:biotinylated firefly luciferase).

Once the above reactions are complete, the amount of firefly luciferase bound may be detected by reaction with the appropriate substrates, i.e., adenosine triphosphate and luciferin, in the well known manner. The amount of light generated is directly proportional to the amount of ligand in the sample. The light may be measured conventionally using a photometer and photomultiplier tube. The amount of ligand present in the sample may then be determined by comparison to standard curves prepared by assaying samples having known amounts of ligand in the manner just described.

The following experiments are offered by way of example and not by way of limitation.

<u>EXPERIMENTAL</u>

All percentages are by weight unless otherwise noted.  The following abbreviations are employed:

| | | |
|---|---|---|
| ATP | - | adenosine triphosphate |
| BBS | - | borate buffered saline |
| BQS | - | Triton QS-15 (0.125%) and bovine hemoglobin (1mg/ml) in PBS |
| BSA | - | bovine serum albumin |
| DMSO | - | dimethylsulfoxide |
| EDAC | - | 1-ethyl-3-(3-dimethylaminopropyl) carbodiimide-HCl |
| EDTA | - | ethylenediaminetetraacetic acid |
| HGG | - | human gamma globulin |
| NHSB | - | N-hydroxysuccinimide biotin ester |
| OD | - | optical density |
| PBS | - | phosphate buffered saline |
| PBST | - | PBS with 0.1% Tween® 80 |
| PBSTT | - | PBST with 0.1% Triton® X-100 |
| PBSTB | - | PBS with 0.1% Tween® 80 and 0.1% BSA |
| PRG | - | perennial rye grass |
| QS | - | Triton QS-15 (0.125%) in PBS |
| RT | - | room temperature |
| Tris | - | 50mM Tris, 5mM MgSO$_4$ 0.5mM EDTA, pH 7.8 |
| Tris-BSA | - | Tris with 0.1% BSA |

<u>Materials and Methods</u>

(a)  Firefly luciferase and BSA (Lot No. 53F-0255) were obtained from Sigma Chemical Co., St.

Louis, MO. Biotinylated goat anti-(human IgE) was obtained from Tago, Burlingame, California. Avidin was obtained from Vector Laboratories, Inc., Burlingame, CA. Allergens were obtained from Hollister-Stier, Spokane, Washington.

(b) Standard polypropylene test tubes were prepared for use in the specific IgE assays as follows. Gluteraldehyde (100μl, 1% in 0.1M carbonate buffer, pH 9.0) was added to each tube and incubated for 1 hour at 56°C. After cooling for 10 min. at room temperature, the gluteraldehyde solution was aspirated and the tubes were washed 2x with tap water and the desired allergen (100μl, diluted 1:8 in PBS with 0.025% glutaraldehyde) was incubated in each tube at 4°C overnight. The allergens were as follows: birch or PRG.

(c) Standard polypropylene test tubes for use in the total IgE assays were prepared as follows. Glutaraldehyde (100μl, 1% in 0.1M carbonate buffer, pH 9.0) was added to each tube and incubated at 56°C for 1 hour. After cooling for 15 minutes at room temperature, the glutaraldehyde solution was aspirated, and the test tubes were washed 2x with saline (2ml, 0.9%). Anti-IgE (100μl, Tago biotinylated anti-IgE (Lot No. 040201) diluted 30:1 in PBS with 0.0125% glutaraldehyde) was incubated in each tube at 4°C overnight. Prior to incubating with the patient samples, the tubes were washed 3x with 1 ml PBST.

(d) Biotinylated firefly luciferase was prepared as follows:

1. To 200μl of 0.828 mg/ml of firefly luciferase in PBS was added 1.6μl of 10mM NHSB in DMSO. After 2.5 hours at 4°C, the luciferase was passed over a $P_2$ column. The fractions exhibiting luciferase activity referring to both the intrinsic activity of the enzyme and the ability of the enzyme to catalyze a bioluminescent reaction when employed in the assay of the present invention were pooled. The pool was made 0.2% with ovalbumin in an effort to stabilize the dilute enzyme. This preparation did not last, as the enzyme activity quickly died out. Enzyme activity was assessed by reacting the preparation with immobilized avidin and thereafter exposing the reaction product to luciferin and ATP and measuring the light generated. The "observed" enzyme activity thus included the effect of conjugation on the intrinsic activity of the enzyme as well as the ability of the conjugate to bind the avidin.

2. To 150μl of 12.5μM luciferase solution in PBS was added 2.49 and 7.47 μl of 1mM NHSB in DMSO and 50 μl of BBS pH 8.4 After 2 hours at 4°C, the enzyme active fractions were collected after passage over a $P_2$ column. BSA was added to a final concentration of 1 mg/ml. The lesser amount of NHSB gave a higher initial activity. Within a week, however, activity disappeared.

3. BBS (100μl, pH 8.4), 300μl of 0.83 mg/ml firefly luciferase and 2.49μl of 4mM NHSB in DMSO were combined and left at 4°C for 16 hours. BSA (4mg) was added and the mixture passed over the $P_2$ column. No activity was detected.

4. To 204μl of firefly luciferase in 0.1 M $PO_4^{--}$, 0.1 M NaCl, pH 7.6, was added 2 μmoles of ATP in 295 μl of the same buffer. NHSB (20 nmoles in 2μl DMSO or 40 nmoles in 4μl) was then added and the mixture left at 4°C for 2.5 hours. The mixtures were passed over a $P_2$ column and the fractions displaying activity were pooled. The preparations lost activity after about three weeks.

5. An attempt was made to conjugate biotin through the carboxyl groups of the firefly luciferase. To 204 μl of 2.45 mg/ml firefly luciferase in $PO_4^{--}$ buffer and 2μmoles of ATP was added a mixture of 500 nmoles of biotin-hydrazide and 3.5 μmoles of EDAC in 296μl of phosphate buffer. The mixtures were left to incubate at room temperature for 15 hours. After leaving the mixture overnight, the enzyme activity of both preparations was assayed. Neither preparation retained luciferase activity.

6. To 102µl of 2.45 mg/ml firefly luciferase in 0.1M $PO_4^{--}$, 0.1M NaCl, pH 7.6, buffer was added 148µl of BBS, pH 8.4, containing 4 µmoles of ATP. NHSB (2µl, 4.26 µg/ml) in DMSO was added, and the mixture left at room temperature for 2 hours. The mixture was dialyzed against PBS, and azide (0.02%) and human hemoglobin (1.5 mg) were added. Enzyme activity was lost after a week.

7. To 102µl of 2.45 mg/ml firefly luciferase in 0.1 M $PO_4^{--}$, 0.1M NaCl, pH 7.6, buffer was added 148µl of BBS, pH 8.4, containing 8 µmoles of ATP. NHSB (2µl, 4.26 µg/ml) in DMSO was added and the mixture left at room temperature for two hours. The mixture was dialyzed against PBS, and azide (0.02%) was added. Enzyme activity was lost after 4 days.

8. To 102µl of 2.45 mg/ml firefly luciferase in 0.1 M $PO_4^{--}$, 0.1 NaCl, pH 7.6, buffer was added 1µmole ATP in 147 µl of the same buffer. NHSB (20 nmoles) in 2µl DMSO was added, and the mixture left overnight at 4°C. This enzyme preparation retained activity for about one month (4-5 weeks).

9. The preparation described in no. 8 was repeated. Enzyme activity was retained for about 3 weeks until used up in the experiments described below.

10. The preparation described in no. 8 was repeated, except that 40 nmoles of NHSB were added. This preparation retained the highest enzyme activity. The preparation was kept for three weeks until used up in the experiments described below and did not lose much activity in that period.

11. The preparation described in no. 10 was repeated, except that 0.153 mg/ml of firefly luciferase

14

was used. The preparation did not display substantial activity.

12. The preparation described in no. 11 was repeated, except that 20 nmoles and 40 nmoles of NHSB were used. The preparations did not display substantial activity.

13. To 0.3 nmole of firefly luciferase in 0.1 M $PO_4^{--}$ buffer (as described above) and 1 μmole ATP was added 5, 2.5, 1.25 and 0.6 nmole NHSB. All activity present was lost during dialysis, even in the presence of BSA.

14. To 0.2 ml of 832 μg/ml firefly luciferase in 0.25 M sodium arsenate, pH 7.5, was added 1 μmole of ATP and 22 or 40 nmoles of NHSB in 1.1 or 2 μl DMSO. After incubating at 4°C overnight, the preparations were assayed. Both displayed substantial activity and were used in the assays described below.

(e) Anti-HGG (I.9881) and HGG (I.4506) were obtained from Sigma, St. Louis, Missouri.

(f) Specific IgG against PRG was isolated as follows: PRG was diluted 1:2 in PBS and contacted with AffiGel 10 (Bio-Rad Laboratories, Inc., Richmond, California). After incubating overnight, the AffiGel beads were washed until the eluate had an $OD_{280} <$ 0.010. The IgG portion of a patient sera activity against PRG was isolated by passing the sodium sulfate cut (immunoglobulin fraction) through a sieving column (Bio Gel A1.5 m, Bio-Rad). The IgG fraction was passed through a PRG immunoadsorbent column. The column was washed until the $OD_{280}$ was less than 0.010. The bound IgG was eluted with 0.3M acetic acid and immediately neutralized with Tris base (Sigma). The amount of IgG in the eluate was quantitated by total IgG assay. The specific IgG was able to bind to PRG, as exemplified below.

15

## Results

### Total IgG assay

We treated polypropylene tubes (12 x 75mm) with 1% glutaraldehyde in 0.1M carbonate buffer, pH 9.0, for 90 min. at 56°C. We cooled them to room temperature for 20 min. We washed them once with water. We added to each tube 100 µl of 1:40 dilution of goat anti-human gamma globulin (HGG) serum in PBS containing 0.025% glutaraldehyde. We incubated the tubes overnight at 4°C. We washed them three times with 1 ml of PBST. We added 100 µl of serum dilution done in PBST (1:100,000). As a control, we used varying known amounts of HGG. We incubated the tubes at 37°C for 55 min. We washed the tubes three times with 1ml of PBST. We added 100 µl of 1:400 dilution in PBST of biotinylated anti-HGG. We waited 10 min. at room temperature before aspirating the supernates. We added 100 µl of 20 µg/ml avidin in PBST. After 10 min. at room temperature, we aspirated the supernates. We added 100 µl of 1:2000 dilution of biotinylated luciferase in PBST. After 15 min. at room temperature, we washed them two times with 1 ml PBST and once with 0.5 ml of Tris buffer. We added 200 µl of $10^{-5}$ M of luciferin - ATP in Tris buffer. We read the light emitted from duplicate tubes and averaged the readings. The results were as follows:

| Reference | | Patients | |
|---|---|---|---|
| HGG(µg/ml) | Light | Number | IgG (mg/ml) |
| 5 | 17.74 | 1 | 18.6 |
| 1 | 12.55 | 2 | 20.9 |
| 0.2 | 7.93 | 3 | 17.3 |
| 0.04 | 2.80 | 4 | 12.9 |
| 0.008 | 1.57 | 5 | 8.0 |
| 0.0016 | 1.13 | | |

Specific IgG assay

Polypropylene tubes were derivatized and washed as indicated in the total IgG assay described above. We coated the tubes with a 1:8 dilution of perennial rye grass (PRG) in PBS containing 0.025% glutaraldehyde (100 µl per tube), and left them overnight at 4°C. After washing three times with 1 ml of PBST, we added 100 µl of dilution in PBST of patient serum. As a reference, we used dilutions of known amounts of affinity purified anti-PRG IgG. We incubated for 2.75 hrs at room temperature, and washed the tubes three times with 1 ml of PBST. We added 100 µl of 1:400 dilution in PBST of biotinylated goat anti-HGG, and waited 6.3 hrs at room temperature. We aspirated the supernates, and added 100 µl of 20 µg/ml avidin in PBST. After 10 min. at room temperature, we aspirated the supernates. We added 100 µl of 1:2000 dilution of biotinylated firefly luciferase in PBST. After 10 min. at room temperature, we washed them twice with 1 ml of PBST and once with 0.5 ml of Tris buffer. We added 200 µl of $15^{-5}$ M luciferin-ATP in Tris. We read the light from duplicate samples, and averaged the readings. The results were as follows:

| Reference | | Patient | |
|---|---|---|---|
| anti-PRG (µg/ml) | Light | Number | anti-PRG (µg/ml) |
| 0.678 | 11.55 | 1 | 4.27 |
| 0.136 | 5.46 | 2 | 32.0 |
| 0.0271 | 2.36 | 3 | 8.88 |
| 0.00542 | 1.60 | 4 | 41.6 |
| | | 5 | 5.9 |

Specific IgE assay

Tubes were treated as indicated in the above examples and coated with 1:8 dilution of birch or PRG in PBS containing 0.025% glutaraldehyde. The tubes were placed at 4°C overnight, and washed three times with 1 ml of PBST. Then 100 µl of serum dilutions in PBST were added. As a control, known amounts of anti-birch antiserum diluted in PBST were used. All tubes were incubated at 37°C for 45 min. After three PBST washes, 100 µl of 1:100 dilution of biotinylated goat anti-human IgE in PBST was added. The tubes were incubated for 45 min. at 37°C. The supernates were aspirated. Avidin (40 µg/ml) was added. After aspirating the supernates following a 10 min. incubation at room temperature, 100 µl of 1:2000 dilution of biotinylated luciferase in PBST was added. The tubes were incubated at room temperature for 10 min. The tubes were then washed twice with 1 ml PBST and once with 0.5 ml Tris buffer. After addition of 200 µl of $10^{-5}$ M substrate, the light generated from duplicate samples was recorded and averaged. The results were as follows:

| Reference | | | Patient | |
|---|---|---|---|---|
| anti-birch IgE (PRU) | Light | | Number | IgE (PRU) |
| 0.350 | 1.985 | | 1 | 42.0 |
| 0.175 | 1.928 | | 2 | 15.5 |
| 0.0875 | 1.861 | | 3 | 1.33 |
| 0.04375 | 1.717 | | | |
| 0.0218 | 1.639 | | | |
| 0.0109375 | 1.542 | | | |

Total IgE assay

Polypropylene tubes were treated with glutaraldehyde at 56°C for 65 min. The tubes were cooled for 30 min. at room temperature and washed twice with water. Then 100 µl of 1:30 dilution of goat anti-human IgE in PBS containing 0.025% glutaraldehyde was added. The tubes were incubated overnight at 4°C. After 3 PBST washes, 100 µl of serum dilution of PBST was added. As a reference, known dilutions of IgE in serum were used in parallel. The tubes were incubated for 90 min. at 37°C, and washed three times in PBST. We added 100 µl of 1:200 dilution in PBST of biotinylated anti-IgE, and incubated at 37°C for 90 min. After aspirating the supernates, 100 µl of 40 µg/ml avidin in PBST was added. After 15 min. at room temperature, the supernates were aspirated. Then, 100 µl of 1:2000 dilution of biotinylated luciferase was added. After a 15 min. incubation at room temperature, the tubes were washed and the light read. The results were as follows:

| Reference | | | Patient | |
|---|---|---|---|---|
| IgE (IU/ml) | Light | | Number | IgE (IU/ml) |
| 100/ml | 3.67 | | 1 | 878 |
| 50/ml | 2.66 | | 2 | 819 |
| 20/ml | 1.80 | | 3 | 289 |
| 7.5/ml | 1.58 | | 4 | 358 |
| 2.5/ml | 1.22 | | 5 | 566 |
| | | | 6 | 519 |
| | | | 7 | 110 |
| | | | 8 | 105 |
| | | | 9 | 1217 |
| | | | 10 | 2893 |

The above results were compared against measurements taken with a commercial IgE assay kit (PRIST, Pharmacia Diagnostics, Piscataway, NJ), and a 98% correlation was established.

### Comparison of luciferase and alkaline phosphatase labels

The following assays were performed to compare the sensitivity of assay of the present invention using luciferase as the label with an identical assay protocol except that alkaline phosphatase was used as the label.

Glutaraldehyde (1%) in carbonate buffer (100µl, pH 9.3, 0.1M) was added to standard polypropylene tubes and left for 60 minutes at 48°C. After washing twice (without cooling) with 0.9% saline (2ml), 100µl of either anti-HGG (Sigma) or anti-IgM (Sigma) diluted 1:40 in PBS with 0.025% glutaraldehyde were added to the tubes, and the tubes were incubated at RT for 90 minutes. After washing three times with QS (1ml), patient serum (100µl) diluted in BQS was added to each tube and allowed to incubate for 90 minutes at 37°C. Biotinylated anti-IgG (1:200) or anti-IgM (1:400) in BQS was then added and allowed to incubate for 60 minutes at 37°C. After aspirating the serum, avidin in BQS (20µg/ml, 100µl) was added to each tube and incubated for 15 minutes. Biotinylated luciferase (100µl), 1:4000 dilution in BQS) or biotinylated alkaline phosphatase (Sigma, 71F-3928, 1:500 dilution in BQS) were next added to each tube. After incubation for 15 minutes at RT, the tubes were washed twice with QS (1ml) and once with Tris-BSA (0.5ml).

To the tubes which received the biotinylated luciferase were added 200µl of $10^{-5}$M luciferin-ATP in Tris buffer. The light emission from these tubes was then read immediately, and the results recorded. To the tubes which received the biotinylated alkaline phosphatase were added substrate (300µl). After sitting at room temperature for one hour, the tubes were read in a spectrophotometer and the results recorded. The results were as follows:

| Concentration (μg/ml) | IgG | | IgM | |
|---|---|---|---|---|
| | Luciferase[1] | Alkaline[2] Phosphatase | Luciferase[1] | Alkaline[2] Phosphatase |
| 1.0 | 23.6 | 1.33 | 17.58 | 1.16 |
| $2 \times 10^{-1}$ | 14.16 | 0.75 | 3.82 | 0.30 |
| $4 \times 10^{-2}$ | 4.76 | 0.30 | 1.79 | 0.16 |
| $8 \times 10^{-3}$ | 1.34 | 0.08 | 0.78 | 0.04 |
| $1.6 \times 10^{-3}$ | 0.58 | 0.03 | 0.75 | 0.07 |
| $3.2 \times 10^{-4}$ | 0.42 | 0.03 | 0.53 | 0.07 |
| $6.4 \times 10^{-5}$ | 0.46 | 0.08 | 0.83 | 0.10 |
| $1.28 \times 10^{-5}$ | 0.55 | 0.14 | 0.66 | 0.06 |
| $2.5 \times 10^{-6}$ | 0.46 | 0.03 | 0.64 | 0.05 |
| Control | 0.22 | 0.01 | 0.18 | 0.00 |

1.  Reading in light units measured on photometer.  Average of three measurements.

2.  Read is ΔOD at 405 nm.  Average of three measurements.

As can be seen from these results, the luciferase label is sensitive to a concentration of about $1.6 \times 10^{-3}$ μg/ml, while the alkaline phosphatase label is sensitive only to $4 \times 10^{-2}$ μg/ml.  The difference in sensitivity is thus about 25 fold, with the absolute sensitivity of the luciferase in the range of 1ng/ml.

Although the foregoing invention has been described in some detail by way of illustration and example for purposes of clarity of understanding, it will be obvious that certain changes and modifications may be practiced within the scope of the appended claims.

CLAIMS

1.   A heterogeneous immunoassay for the determination of a ligand in a liquid medium, said immunoassay including the steps of:

reacting the liquid medium with a first receptor capable of binding the ligand, said first receptor being immobilized on a solid phase;

separating the solid phase from the medium;

exposing the solid phase in a predetermined order to (a) a biotinylated second receptor capable of binding the ligand, (b) avidin, and (c) biotinylated luciferase, whereby the luciferase is bound to the solid phase in preparation to the amount of ligand in the liquid medium;

separating the bound luciferase on the solid phase from the unbound biotinylated luciferase remaining in the liquid medium;

determining the amount of bound or unbound luciferase to substrate and measuring the emitted light.

2.   A heterogeneous immunoassay as in claim 1, wherein the solid phase is exposed first to the biotinylated second receptor and after separation from the second receptor, exposed simultaneously to the avidin and biotinylated luciferase.

3.   A heterogeneous immunoassay as in claim 1, wherein the solid phase is exposed simultaneously to preselected amounts of the biotinylated second receptor, avidin and biotinylated luciferase.

4.   A heterogeneous assay as in claim 1, where in the solid phase is exposed first to the biotinylated second receptor and thereafter to preselected amounts of the avidin and the biotinylated luciferase which have been pre-reacted.

5.     A heterogeneous immunoassay as in any one of claims 1 to 4, wherein the biotinylated luciferase is firefly luciferase which, after biotinylation, retains at least 50% of the activity of unmodified firefly luciferase.

6.     A heterogeneous assay as in any one of claims 1 to 5, wherein the ligand is IgE, the first receptor is anti-(IgE), and the second receptor is an allergen, whereby IgE to the particular allergen may be measured.

7.     A heterogeneous assay as in any one of claims 1 to 5, wherein the ligand is IgE and both the first and second receptors are anti-(IgE), whereby total IgE response may be measured.

8.     A heterogeneous assay as in any one of claims 1 to 7, wherein at least one detergent is added to the assay medium to inhibit background luminescence.

9.     A specific binding assay of the type wherein biotin is bound onto a solid phase in direct or inverse proportion to the amount of ligand in a sample, said assay characterized by exposing the solid phase having bound biotin to preselected amounts of avidin and biotinylated firefly luciferase so that the avidin and biotinylated luciferase combine with the biotin on the solid phase, wherein said biotinylated firefly luciferase retains at least 50% of the activity of unmodified firefly luciferase.

0141581

10.     A biotin-firefly luciferase conjugate characterized by retention of at least 50% of the activity of the unmodified luciferase.